# EUROPEAN PATENT APPLICATION

(11) **EP 0 939 084 A1**
(43) Date of publication of application: **01.09.1999**
(21) Application number: 98200423.6
(22) Date of filing: 11.02.1998
(51) Int. Cl.: C07K 14/47

(54) **An estrogen binding proteinaceous substance, its possible role in estrogen action, and potential use.**

(71) Applicant: Rao, Ramanath B., 1081 HK Amsterdam (NL)
(72) Inventor: Rao, Ramanath B., 1081 HK Amsterdam (NL)
(74) Representative: Smulders, Theodorus A.H.J., Ir.

(57) **Abstract**

The invention relates to an estrogen binding proteinaceous substance. The substance is different from the classical cytosol or nuclear ER (type I and II) in a number of characteristics. The novel estrogen binding substances according to the invention will be collectively referred to as estrogen binding protein (EBP).

The invention provides a murine estrogen binding proteinaceous substance or a mammalian equivalent thereof, the murine substance comprising a subunit having a molecular weight of about 61-67 kD as analyzed by SDS-PAGE.

In its active form this proteinaceous substance usually appears as a heterodimer, whereby one subunit of the murine substance is the 61-67 kD proteinaceous substance and the other subunit is a 17-22kD proteinaceous substance, whereby the resulting heterodimer has a molecular weight of about 81-84 kD as measured by gel electrophoresis.

The genes encoding EBP and antibodies directed against EBP are also part of the invention.

## Description

The invention relates to the field of steroid hormones and their binding to various receptors and other binding substances. It relates in particular to binding substances for steroid hormones that have an affinity for estrogen.

Estrogen induces a plethora of actions (1-4). It is generally accepted that estrogenic action is mediated by specific estrogen receptors (ER) present in estrogen target tissues (5-9). However, the majority of estrogen related physiological functions can not be explained on the basis of action mediated through the ER. The vagina is the most sensitive estrogen responsive organ in the female. The presence of ER and the role of ER in estrogenic action on the vagina has never been well determined. Only a few publications have appeared in the literature on detection of ER in the vagina and the results have sofar not been consistent. Also, there is little information about the presence of ER in the central nervous system in which estrogen significantly influences the behavioral and biochemical consequences which regulate for instance the well being of the female (10-13). Similarly the role of ER has not been well characterized in a variety of other estrogen related functions such as the negative (LH and FSH) and in particular the positive (prolactin) feed back mechanism of estrogen at the hypothalamic-pituitary level (14-19). Only recently, dopamine has been shown to regulate estrogen responsive elements, but most strikingly the action appears not to be mediated through the estrogen receptor but through an unknown mechanism (20). Furthermore there is hardly any publication in which ER has been related to the potent action of alleviation of chorea by estrogen in patients treated with neuroleptics, in the mechanism of action of estrogen as an antipsychotic agent, the role of estrogen in striata nigra in Alzheimer's or Parkinson diseases. Similarly the role of ER and estrogens in the protective action against heart infarct in premenopausal women, immune protective action and the role in bone density maintenance is largely undetermined(21-29). The studies on correlation between the presence of ER in the estrogen dependent cancers and the response to endocrine therapy have shown that there may be other additional mechanisms by which estrogen induces its action (30). In this context, the role of estrogen in carcinogen induced tumors is also not clear (31-36). We have shown that there is substantial evidence to demonstrate that the classical ER is not involved in the induction of estrogenic action of some compounds which are as potent as the natural estrogen, estradiol-17β (37,38). Recently, a book has been published arguing strongly the point that there may be a "non-genomic" alternative mechanism of steroid hormone action mediated by mechanisms other than via the classical receptors (39).
Thus there is a clear need to investigate the existence of alternative mechanisms for estrogen action and to investigate the presence of other types of estrogen binding substances for a complete understanding of the multi-actions of estrogens, so that the many estrogen related disorders in mammals can be better treated.
The present invention provides a novel estrogen binding proteinaceous substance which has been demonstrated to be related to some of the unexplained estrogen related phenomena in mammals.
According to the invention there is disclosed another form of estrogen binder which is different from the classical cytosol or nuclear ER (type I and II) in a number of characteristics. The novel estrogen binding substances according to the invention will be collectively referred to as estrogen binding protein (EBP) An overview of the substantial amount of data collected on EBP is provided in table 1.
Thus the present invention provides a murine estrogen binding proteinaceous substance or a mammalian equivalent thereof, the murine substance comprising a subunit having a molecular weight of about 61-67 kD as analyzed by SDS-PAGE. In its active form this proteinaceous substance usually appears as a heterodimer, whereby one subunit of the murine substance is the 61-67 kD proteinaceous substance and the other subunit is a 17-22kD proteinaceous substance, whereby the resulting heterodimer has a molecular weight of about 81-84 kD as measured by gel electrophoresis.
The exemplified substance according to the invention can be further characterized in that it has an iso-electric point of about 4.35 and/or that it shows a single peak at 4.8 S in a sucrose density gradient centrifugation. Another form in which the estrogen binding proteinaceous substance according to the invention has been found is a form comprising a further subunit of about 61-67kD respectively as measured by gel electrophoresis.
Preferably the estrogen binding proteinaceous substance retains estrogen binding activity in the presence of the reducing agent DTT.
Preferably the estrogen binding proteinaceous substance does not show specific binding to anti-estrogen receptor antibodies.
Preferably the estrogen binding proteinaceous substance has serine phosphatase activity.
The estrogen binding substances according to the invention, starting with the 61-67 kD subunits, the 17-22 kD subunits, the combined subunit forming the 84 kD substance or the heterodimer thereof with the further 61-67 kD subunit can be provided as they are exemplified here, but these subunits may vary in their molecular weight when found in other species than in the mouse. These other mammalian EBP's are considered to be part of this invention. Since sequencing of the gene(s) encoding EBP is routine work nowadays for which a simple handbook like Maniatis et al. is available and since having the sequence in hand finding other related sequences using for instance PCR is also a routine skill in the art, these other genes and proteins based thereon are also considered part of the present invention. Antibodies to EBP are also part of the invention. Muteines in which parts of the EBP have been deleted or mutated are also part of the present invention. Knowing the nucleic acid sequences or the amino acid sequences it will be feasible to identify regions which can be deleted or modified without significant influence on the relevant activity of EBP. It will also be possible to design mutations which increase or decrease the stability of the EBP as wanted. These techniques are all within the skill of the art and thus within the same inventive concept. Furthermore the uses as disclosed herein are considered to be part of the invention, as are the uses of for instance the antibodies or the antagonists that can be produced using the present invention.
The invention will be illustrated and explained in greater detail in the following detailed description.

### DETAILED DESCRIPTION.

### ISOLATION AND PURIFICATION OF EBP:

At initial stages a variety of methods and tissues were screened for the isolation and purification of EBP prior to arriving at the definitive procedure. The final developed procedure for the isolation and purification of EBP in the **mouse fetal liver** (19 days) is as follows. All procedures were performed on ice in the cold room (4°C). The tissue was extensively washed with cold saline, dried on tissue paper, weighed, cut into small pieces and suspended in buffer (50 mM sodium acetate plus 0.4 pyridine, adjusted to pH 6.5 by careful addition of 12.5 N formic acid). The volume of buffer used is three times the weight of the tissue. The tissue suspension was homogenised with an ultra-Thurax homogenizer three times, each pulse was for 10 seconds. Between pulses the suspension was cooled for 2 minutes on ice. The homogenate was centrifuged at 2500 rpm for 15 minutes and the separated supernatant was centrifuged again at 35,000 rpm (100,000 x g) at 4°C for 60 minutes using an angle rotor in a Beckman ultracentrifuge. The supernatant was separated and the pH thereof was adjusted to 5.6 with the addition of formic acid and frozen. The frozen supernatant was thawed slowly in the cold room, centrifuged at 2500 rpm for 15 minutes at 4°C to remove the copious precipitated material. The separated supernatant was frozen and the procedure of freezing, thawing and centrifugation to remove the precipitated material was repeated twice. The pH of the final collected supernatant was adjusted to 6.5 and it was concentrated under nitrogen pressure using a membrane filter (cut off value 10,000 dalton, Amicon). The original supernatant was concentrated by this procedure at least by a factor of 6. Two ml samples of the concentrated supernatant were further fractionated using an FPLC gel filtration column (Superdex 200-Pharmacia) at a flow rate of 1 ml/min. Aliquots (10 µl) from each fraction were tested for estrogen binding activity. The assay procedure for estimating the estrogen binding activity is given below. The fractions containing the peak estrogen binding activity which usually amounted to 12 to 14 fractions (12-14 ml) were pooled. Peak fractions containing estrogen binding activity from various runs were collected, pooled and the liquid volume was reduced as before using membrane filters. The concentrated sample was once again run on FPLC gel filtration column, this time at a flow rate of 0.5 ml/min and each fraction of 1 ml. The peak fractions containing estrogen binding activity measured using 5 µl from each fraction was pooled, concentrated, dialysed using membrane bags against double distilled water. The resulting dialysed fraction was distributed in small volumes containing 188 µg of protein per tube, the tubes were lyophilized and stored at -25°C.

### ASSAY PROCEDURE FOR ESTROGEN BINDING ACTIVITY:

In brief, the procedure is as follows. The protein sample in usually around 5-20 µl containing about 2.5 to 10 µg of protein in duplicate was mixed with 0.5 ml of buffer (25 mM Tris-HCl, pH 7.5 containing 150 mM NaCl) and incubated for 45 minutes at room temperature following the addition of tritium labelled estradiol-17β (10 µl, 50,000 dpm, 92 Ci/mmol). Simultaneously tubes in duplicate without any added protein sample were set up as control. At the end of the incubation the tubes were put in an ice bath for 15 minutes to cool to the ice bath temperature. 0.5 ml of charcoal solution (0.05% heat activated charcoal plus 0.05% bovine serum albumin in tris buffer) was added, mixed and incubated further for 10 minutes. The tubes were then centrifuged at 2500 rpm for 15 minutes at 4°C to pellet the charcoal. 0.5 ml from each tube was carefully withdrawn without disturbing the charcoal pellet at the bottom of the tube and the radioactivity was measured following mixing with the scintillation cocktail. The net protein bound radioactivity was calculated after deducting the background counts based on the tubes without any added protein.

### SPECIFIC BINDING OF ESTRADIOL-17β TO EBP:

Investigations of the specificity of estrogen binding activity showed that the EBP is highly specific for estradiol-17β. The results given in table 1 show that estradiol-17β binding to EBP was not inhibited by any of the non estrogens tested (testosterone, dehydrotestosterone, progesterone, RU5020, corticosterone, cortisol) at 200 times the concentration of the radiolabelled estrogen. On the other hand the natural estrogens estrone and estriol effectively competed with ³H-estradiol-17β for binding sites and significantly inhibited the ³H-estradiol-17β binding. However, the estradiol-17β antipode, estradiol-17α, had no inhibitory effect on ³H-estradiol-17β binding even at 300-fold the concentration of the radiolabelled estrogen. There was a dose response relationship between the concentration of the natural estrogens used and the ³H-estradiol-17β binding. The relative binding activities of the competing estrogens were calculated (at the 50% reduction level) and compared to non-labelled estradiol-17β competing potency. The calculated values for the natural estrogens are as follows:
**Estradiol-17β - 100%, estrone - 80%, estriol - 37%.**

### SYNTHETIC ESTROGENS AND EBP:

Although EBP showed high specificity in that only non-labelled natural estrogens competed with the ³H-estradiol-17β, the potent non-labelled synthetic estrogen diethylstilbestrol did not inhibit the ³H-estradiol-17β binding significantly even at 200-fold concentration of the radiolabelled estradiol-17β. This was also supported by the finding that ³H-diethylstilbestrol does not bind to EBP. Another commonly used synthetic estrogen, ethinylestradiol behaved in a similar fashion as diethylstilbestrol and had no inhibitory action on ³H-estradiol-17β binding.

### SYNTHETIC ANTI-ESTROGENS AND EBP:

Tamoxifen and 4-hydroxytamoxifen, the parent and the active metabolite of antiestrogen respectively, also had no significant inhibitory effect on ³H-estradiol-17β binding to EBP. A similar result was obtained using radiolabelled tamoxifen aziridine which is an effective affinity labelling compound.

These results demonstrate that the EBP differs from the classical ER to which diethylstibestrol, tamoxifen, 4-hydroxytamoxifen and tamoxifen aziridine are known to bind.

### BIOFLAVONOIDS AND EBP:

Competitive binding assays performed to see whether bioflavonoids such as querecetin, rutin, luteolin and hespertin, showed that none of them at 200- to 400-fold concentrations of the radiolabelled estradiol-17β inhibited the ³H-estradiol-17β binding to EBP to any significant extent. These compounds have been shown to compete with ³H-estradiol-17β for binding sites on type II ER (40,41).

### IMMUNOSUPPRESSANTS, NEUROLEPTICS AND CALMODULIN, AND EBP:

A number of other agents such as immunosuppressants (cyclosporin, FK506), neuroleptics (haloperidol, pimozide, reserpidine) and calmodulin which have some relationship to estrogenic action **are not known to be estrogens** nor is there any information available to suggest that they interact with ER type I or II. Interestingly these compounds behaved similar to estradiol-17β in that they potentiated ³H-estradiol-17β binding to EBP in lower concentrations and inhibited at higher concentrations. Similar tests carried out with dopamine, oxytocin and prolactin which also have a relationship to estrogenic action did not influence ³H-estradiol-17β binding to EBP. This is the first demonstration that immunosuppressants and neuroleptics influence the estrogen binding, suggesting that they may share some common mechanism and/or action.

### STABILITY OF EBP:

The purified EBP was tested for its ability to withstand changes in temperature on the basis of its estrogen binding activity and its mobility in gel electrophoresis. The sample in estrogen binding assay buffer was incubated at various temperatures from 30 to 90 degrees for 10 minutes, cooled to ice bath temperature and tested for estrogen binding and mobility in gel electrophoresis. Estrogen binding activity tested at room temperature did not significantly deviate following the incubation at various temperatures, suggesting that the purified EBP is quite heat stable. The results may also indicate that the purified EBP is not contaminated with any protein hydrolysing protease. This result was confirmed by the finding that the EBP incubated at different temperatures migrated to the same level following gel electrophoresis. A similar conclusion was also reached following the treatment of the sample in buffer for 120 seconds in the microwave oven at maximum power. The purified EBP appears to be remarkably stable.

### EFFECT OF SULFHYDRYL REAGENTS:

The purified EBP does not require the reduction of the sulfhydryl groups for binding to estradiol-17β. There was no significant difference in estradiol-17β binding to EBP in the presence or absence of sulfhydryl reagents dithiotreitol (DTT) or β-mercaptoethanol, at various concentrations (0.001 to 5.0 mM). This property of EBP differs from the type II ER which is highly sensitive to the presence of DTT (40-41,43).

### AFFINITY OF ESTROGEN BINDING AND ESTIMATION OF NUMBER OF ESTROGEN BINDING SITES ON EBP:

Estradiol-17β binding affinity to EBP was analyzed in equilibrium binding experiments. In preliminary experiments it was determined that equilibrium of estradiol-17β binding to EBP was reached within 30 minutes at room temperature. Samples of EBP were incubated at room temperature with a series of ³H-estradiol-17β concentrations ranging from 0.125 nM to 16.8 nM and in parallel tubes with the addition of 100 fold molar excess of non-labelled estradiol-17β. Activated charcoal in the presence of albumin was used to separate protein bound from unbound steroid. The radioactive counts in the tubes containing the non-labelled estradiol-17β were subtracted from the counts measured in tubes without the addition of non-labelled estradiol-17β to arrive at the specific ³H-estradiol-17β binding. The binding data at higher molarities of estradiol-17β show a progressive saturation of the binder. The data calculated by the method of least squares indicates that the association constant for the binding action is 0.7 nM. The mean association constant calculated from three experiments was 0.7 ± 0.06 (S.E.M.) nM. The total binding sites calculated for ³H-estradiol-17β at saturation correspond to 0.746 ± 118 pmol/mg of EBP protein.

The various Scatchard plots obtained for the interaction of estradiol-17β and EBP showed an initial positive slope at low molarities of ligand followed by a negative slope at higher estradiol-17β concentrations. This result can not be ascribed as due to non-achievement of equilibrium at the lower concentrations of estradiol-17β, since there was no significant difference in specific binding of samples incubated from 0.5 to 4 hrs at room temperature. One of the explanations for such a cooperative binding observed at low molarity may be the recruitment of new EBP in the presence of estradiol-17β from the sample pool.

### MOLECULAR CHARACTERISTICS OF EBP:

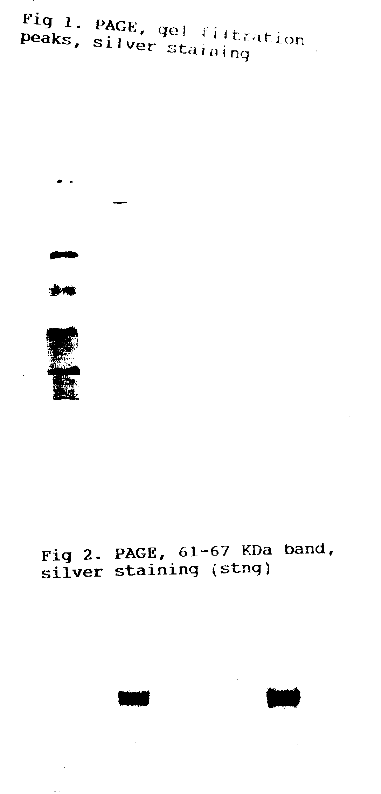

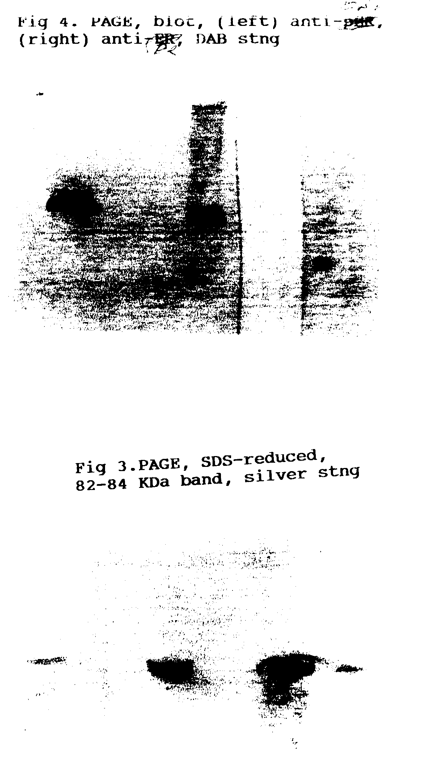

Two distinct bands of proteins were observed on gel electrophoresis in the estrogen binding peak fractions from superose gel filtration procedure (Fig. 1 and 2). The molecular weights of the two bands were about 82-84 KD and 61-67 KD as estimated by comparison with a number of protein standards of known molecular weight run simultaneously in different lanes of the gel electrophoresis. Henceforth, the 82-84 kD band will be referred as 84 kD protein and the 61-67 kD band as 61 kD protein. Areas of gels corresponding to the stained band were cut from other similar gel electrophoreses run and the eluted proteins from the two bands were further analyzed. The protein eluted from the faster moving band 61 kD) on the gel electrophoresis showed no estrogen binding activity while the protein eluted from the 84 KD band showed a high affinity estrogen binding activity. The calculated estrogen binding activity of the protein from the higher molecular weight band accounted for the binding activity detected in the gel filtration fractions. The protein from the 84 kD band was treated with sodium dodecyl sulphate (SDS) and the reducing agent mercaptoethanol and the sample was boiled for 15 minutes. Analysis by gel electrophoresis showed the separation the 84 kD protein into one major distinct band which migrated as a 61-67 kD band and another minor band which migrated in the 17-22 kD region. These analyses established the existence of two subunits of unequal size and demonstrating the 84 kD protein is a heterodimer. This suggests that one or more of the proteins in the 61 kD band observed in the absence of the reducing agent may form a part of the larger 84 kD protein band and the formation of 84 kD band protein may require the binding of the smaller subunit. Frequently, only the major band (61-67 KD) was visible on coomassie blue or silver staining following the SDS and reducing agent treatment (Fig. 3). The smaller unit was not discernable either due to its being lost or its unstainable characteristics. A number of initial attempts made to disassociate and isolate the two heterodimers and re-associate them to form into the original size molecule using standard mild biochemical techniques were not successful suggesting that the two heterodimers are tightly bound. Only drastic methods using a number of agents such as exposure to extreme high urea concentration (8 molar), or chaotropic agents resulted in dissociating the subunits. Analysis of samples following extensive repeated dialysis to remove the dissociating agent showed that the estrogen binding activity is lost and that the two heterodimers can not be re-associated under any of the conditions tested to reform into a single unit of the original size and form with estrogen binding activity.

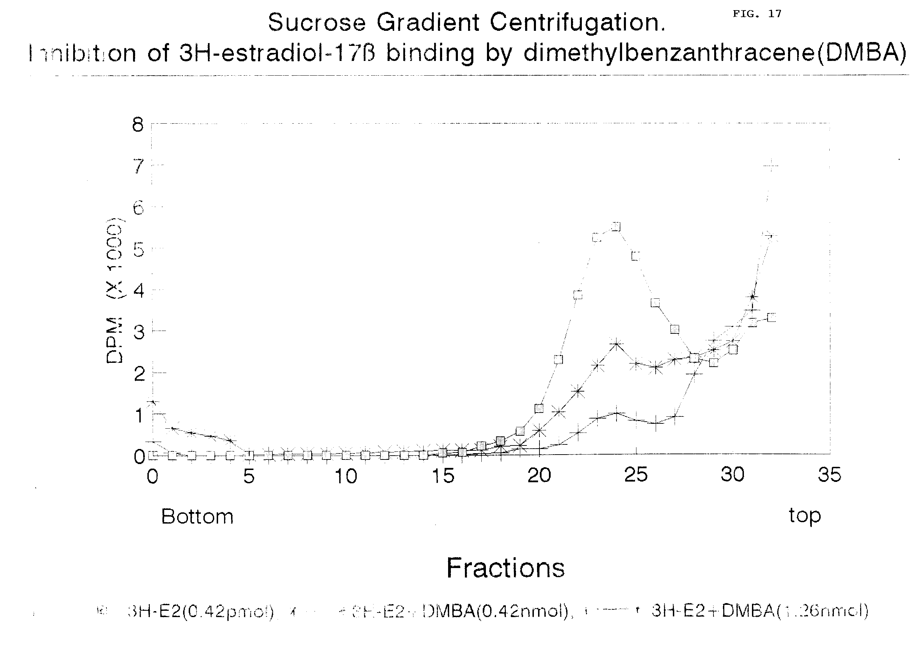

The isolated 84 kD protein was further characterized. The apparent iso-electric point of the molecule is 4.35 and it sediments as a single peak in the 4.8 S region upon sucrose density gradient centrifugation (Fig. 17). The classical ER (type I) from mouse uterus is known to be a monomer of 65,000 Dalton having an iso-electric point around 6.5 (42). Similarly the type II estrogen receptor differs from EBP in having a molecular weight of about 37000 Dalton of which the estrogen binding activity is lost in the presence of the reducing agent DTT (43). As mentioned before estrogen binding to EBP is not significantly affected by the presence of DTT. Recently, the presence of an yet another ER related protein in an exclusive MCF-7 breast cancer clone with a molecular weight of 80,000 has been described (44). The molecule has been shown to be a mutant ER which shares epitopes with ER and reacts with the anti-ER antibody. This mutant ER also differs from the presently discovered EBP based on size and more importantly in its ability to react with the anti-ER (EBP does not react with anti-ER; further details given below).

### INDUCTION AND ISOLATION OF EBP ANTI-BODY:

Concentrated protein (500 µg) from fractions of gel-filtration was loaded on a native 8-25% gradient gel over the whole width of the gel. Standard electrophoresis procedures were followed and the electrophoresis was performed at the room temperature. Following the completion of the electrophoresis run, a strip on either side of the gel was cut off and stained with coomassie brilliant blue. The areas parallel to the 84 and 61 kD stained bands were cut from the unstained gel. The two strips were divided into two equal halves and were used for immunization. The strips were individually frozen with the use of liquid nitrogen, the frozen pieces were each pulverized into a powder and were taken up in Freunds complete adjuvant.

Male guinea pigs were used to induce the antibody. Blood was drawn from each of the animals prior to immunization for the preparation of the pre-immunized serum.

The guinea pigs were injected intradermally with protein from either the 84 kD or the 61 kD band. This was followed by a booster dose of the respective proteins 10 days later. The animals were bled, serum prepared and tested for antibody, and the specificity of the antibody using gel electrophoresis blotting. The antibody fraction was enriched by passing the serum through a column of protein A sepharose and the antibody bound to the column was eluted using acidic (100 mM glycine-HCl, pH 2.5) buffer. The enriched antibody was tested for its potency in serial dilution experiments in which the EBP was precipitated following incubation for either 16 hrs at 4°C or 3 hrs at room temperature. Following this procedure the antibody concentration required for the precipitation of 100% of EBP was determined. The antibody concentration used in subsequent experiments was twice that required to precipitate 100% of the standard EBP protein concentration used in estradiol-17β binding assay.

### PROTEIN BLOTTING AND IMNUNODETECTION:

Polyacrylamide gel electrophoresis has been a widely used technique for the analysis and characterization of protein mixtures. Additionally, the separated protein can be transferred onto the surface of an immobilizing membrane which permits the access of the protein to various probes such as antibody. Nitrocellulose or polyvinylidine difluoride membranes were used for protein transfer from the electrophoretic gel using the standard programmed technique of phastgel system of Pharmacia or Kimtec companies. Following the protein transfer to the membrane from the gel, it was placed in a buffer bath containing gelatin (1%) in phosphate buffered saline and 0.05% tween 20, pH 7.0 (PBST) to block the sites to which protein can still bind on the membrane and incubated in a shaking incubator at room temperature for 60 minutes. Initial trials using bovine serum albumin (1%) to block the non-protein containing sites on the membrane was found to give a dark background and such a procedure was not used further. The membrane was washed thrice with PBST. The washed membrane was then incubated for 16-18 hrs. at room temperature with serum containing polyclonal antibody either prepared against the 84 kD (EBP) or 61 kD protein as the first or the primary antibody at 1:100 dilution in PBST. Next, the membrane was washed by vigorous shaking in the PBST and the procedure repeated two more times. Rabbit anti-guinea pig IgG conjugated with peroxidase at 1:200 dilution in PBST was added and incubated for 60 minutes at room temperature with vigorous shaking. The membrane was washed twice to remove the excess non-reacted second antibody. This was followed by two more washing in phosphate buffered saline. The peroxidase conjugated second antibody which reacted with the first antibody was stained using the deaminobenzadine (DAB) staining kit according to the procedure given by the manufacturer (Pierce). The excess DAB was removed by washing the membrane twice in distilled water. The membrane was allowed to dry at room temperature and the stained band(s) were observed.

### CHARACTERIZATION OF ANTI-EBP ANTIBODY:

Presently, monoclonal antibody against the classical ER is commercially available (Abbott). The antibody has been shown to be highly specific for ER (45). Since the source of EBP is mouse liver only, a polyclonal antibody was prepared against the 84 kD and the 61 kD proteins. The antibody has been tested for reaction with a variety of proteins and was found to be highly specific. It does not react with the ER (type I) protein (Fig. 4). Similar analysis with type II ER also showed no reaction with type II ER (not shown). This finding is further reinforced from the corollary experiment in which it was found that the monoclonal antibodies against the classical ER (Abbott) do not react with the newly discovered EBP. Additional tests showed that the antibody against the EBP does not react with a number of other proteins such as serum albumin (67 kD), alpha feto-protein (67 kD), plasmin (85 kD), fibrinogen (400 kD), α₂-antiplasmin (67 kD) α₂-macroglobulin (300 kD), tissue plasminogen activator (70 kD), urokinase (56 kD), oxytocin (1007 dalton), prolactin (23 kD) and calmodulin (15-19 kD). Furthermore, antibodies against the cell membrane protein p-glycoprotein which regulates the cellular drug uptake, cyclophillin which binds immunosuppressive agents and phospholipase A2 which hydrolyses arachidonic acid from phosphotidyl choline, do not react with EBP.

The rationale for testing the cross reaction with some of the proteins and interaction of EBP with a variety of antibodies will be clear from the information given below.

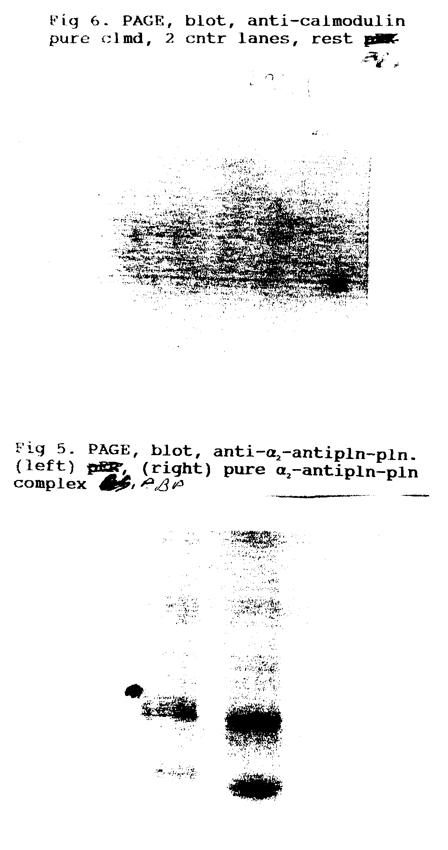

In spite of the high specificity of anti-EBP mentioned above the most interesting and extraordinary finding is that anti-EBP reacts with two relevant and interesting proteins. They are: Alpha₂-antiplasmin-plasmin complex (PAPC) (Fig. 5) and Calcineurin. The relevance of these observations in relation to estrogen action are given below.

### IMPORTANCE OF REACTIONS OF ANTI-EBP ANTIBODY WITH PAPC AND ANTI-PAPC ANTIBODY WITH EBP:

Plasmin (85 kD) is a potent serine protease consisting of a larger A (60-67 kD) and a lighter B (20-25 kD) chains. The fast-acting and physiologically most important inhibitor of plasmin is α₂-antiplasmin (67 kD). A group in Leuven, Belgium has investigated this reaction in depth and has extensively published in this area (46-49). Isoforms of alpha₂-antiplasmin with molecular weights of 65 and 60 kD have also been identified and characterized (50).

Al₂-antiplasmin reacts with plasmin to form a completely inactive 1:1 stoichiometric complex with plasmin through reaction with the B chain of the enzyme which contains the active center. Alpha₂-antiplasmin is known to be the most potent plasmin neutralizing agent which protects against the onslaught of plasmin action in the vascular system. The PAPC appears to be formed through a "tetrahedral intermediate" type reaction resulting in covalent bond for the stabilization of the enzyme-inhibitor complex. The PAPC is devoid of protease or esterase actvitity and the complex cannot be dissociated by denaturing agents such as urea, guanidine HCl, or dodecyl sulfate. The PAPC formation renders this complex antigenically distinct from the precursor molecules and this facilitates PAPC detection, identification and quantitation using immunochemical methods. Most interestingly, the molecular weight of PAPC is about 84 kD and the liver is the major source of α₂-antiplasmin (46,48,50). Both these features are shared with EBP. Polyclonal antibody against the mouse PAPC and the purified mouse standard PAPC material were a generous gift provided through the courtesy of Prof. H.R. Lijnen, Leuven, Belgium. It is indeed remarkable that EBP and PAPC share some common epitopes.

Importantly, it has been repeatedly and extensively reported that estrogen use in prostate cancer treatment and in the contraceptive pills has been associated with cardiovascular complications and thromboembolism. In the case of prostate cancer, more patients died with vascular complications from the use of high dose estrogen than from the prostate cancer. Recently, thromboembolism made headline news when case-control studies disclosed that the third line generation of contraceptives increased the incidence of thromboembolism and myocardial infarction compared with the older second line contraceptives (51,52). Both types of contraceptives contain the potent synthetic estrogen ethinyl-estradiol. Apparently the progestins used in the two combinations appear to play a key role in the regulation of the adverse effect of estrogen (53). Two large cohort long term studies in he past have demonstrated an excess relative risk of myocardial infarction in users of oral contraceptives (54,55). Such a relationship has been reported sporadically by a number of case control studies as well (56-59). However, there is no clear causal evidence explaining how estrogen induces these effects in spite of these numerable reports on estrogen related adverse effect of the vascular system. Very recently, an article in the January 1996 issue of fibrinolysis report that the highest level of PAPC and fibrin degradation products were found in women over 30 years of age who are oral contraceptive users and smokers compared to three other control groups including non-users of oral contraceptive and non-smokers (60). The difference in PAPC level was significant at an impressive p value of 0.0004 compared between the group of oral contraceptive users and smokers with the group of non-users of oral contraceptive pill and non-smokers.

Quantitation of EBP level and the estrogen binding capacity in the blood from such groups may shed light on the important question of the relationship between EBP and PAPC. The development of a detection method to screen those who may be susceptible to the development of such a condition may be useful. Such methods may also be used to monitor the status of contraceptive pill users at regular intervals.

### THE IMPORTANCE OF REACTIONS OF ANTI-EBP ANTIBODY WITH CALCINEURIN AND ANTI-CALCINEURIN WITH EBP:

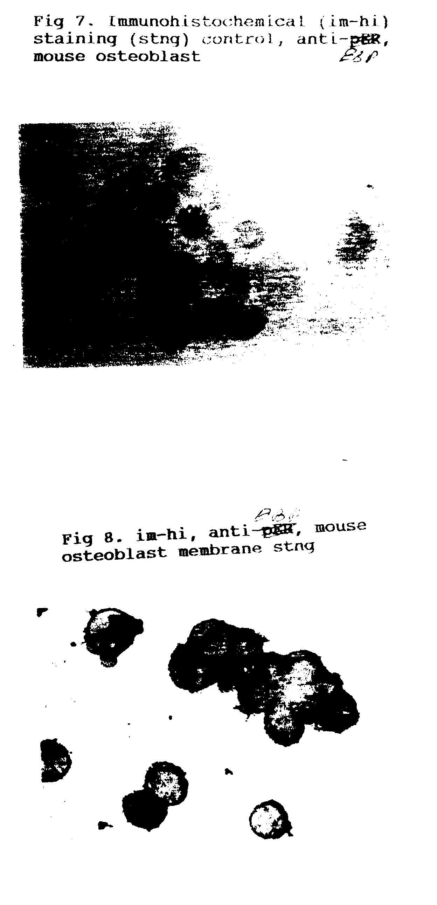

The second protein with which the anti-EBP reacted is Calcineurin, a phosphatase predominantly present in the brain. Calcineurin is also known to be a heterodimer which consist of A (61,000 Da) and B (17-22,000 Da) subunits (61). The B subunit shares some homology with Ca²⁺⁺ sequestering calmodulin and the antibody against the B subunit reacts with calmodulin. Calmodulin and Ca²⁺⁺ ions are required for the phosphatase activity of calcineurin. The anti-EBP does not react with calmodulin (Fig. 6). The interesting relevant fact here is that calmodulin influences estrogen binding to EBP and of equal interest is the finding that EBP has an inherent phosphatase activity similar to calcineurin (further details and discussion on phosphatase action of EBP are presented below in subsequent sections).

Calmodulin plays a role in the phosphorylation of the classical ER and the estrogen binding is increased as a concequence of phosphorylation. This aspect of the classical ER has been extensively investigated by a number of investigators (62, 65). The important messages of their findings have been that calmodulin is required for the phosphorylation of ER, estrogen binding is increased following phosphorylation of ER, dephosphorylation eliminates ER estrogen binding activity and that calmodulin binds to ER. Contrary to these findings with ER the EBP does not require phosphorylation for estrogen binding but EBP itself is a phosphatase. Since estrogen binding to EBP is significantly increased in the presence of Calmodulin a number of attempts were made to investigate whether calmodulin binds to EBP similar as has been observed for the classical ER. One the methods followed was affinity chromatography using calmodulin coupled to sepharose. The fraction containing EBP flowed freely through the sepharose-calmodulin column without retardation or retention of EBP in the column suggesting that calmodulin does not bind to EBP. A similar conclusion was also reached when it was found that radiolabelled calmodulin failed to bind to EBP as opposed to the classical ER (65). The mechanism by which calmodulin potentiates estrogen binding to EBP has to be due to some other mechanism than by binding to EBP.

Two category of protein phosphatases that are investigated extensively are those with specificity for phosphoserine or phosphothreonine and those with specificity for thyrosine residues. Both classes of enzymes have received considerable attention from the academic and pharmaceutical research communities because of their involvement in many cellular processes. Protein tyrosine phosphatases are known to play an important role in cell cycling, bacterial virulence, lymphocyte signalling, and, possibly tumorigenesis (66,67). Protein phosphatases of the serine/threonine class are also involved in many aspects of cellular signalling including immunosuppression, shellfish poisoning and control of cell cycle (68,69). Phosphatases have become attractive targets for drug intervention since many of the cellular functions involving phosphatases are germane to inflammation, transplant rejection and oncology.

As mentioned previously, the phosphatase action of calcineurin requires Ca⁺⁺ ions and calmodulin. The phosphatase assay that is used is frequently based on the release of ³²p phosphate from the phosphoprotein or phosphopeptide. Synthetic peptides have been prepared for such assays (70, 71). The recently published, highly sensitive colorimetric phosphatase assay using Malchite Green and pyrophospate (pp), p-nitrophenylphosphate (pNpp) or DL-phosphotyrosine (pY) and DL-phosphoserine (pS) as substrate was used according to the published procedure (72). There was a dose response relationship between the amount of EBP protein used and the increase in absorbance using either pp or pS as substrate while such a relation was not observed with pY suggesting that EBP may be a serine phosphatase similar to calcineurin. Protein phosphatase (pp-1) was used as a positive control in such an evaluation. Further analysis using pp as substrate showed that EBP phosphatase activity is sensitive to the presence of Ca²⁺⁺ ions. The phosphatase as well as estrogen binding activities of EBP are inhibited in the presence of Ca²⁺⁺ ions. On the other hand the inclusion of calmodulin or the inclusion of EGTA both of which sequester effectively the Ca²⁺⁺ ions, restored both the phosphatase and estrogen binding activities to a level seen in the absence of Ca²⁺⁺ ions. It is possible that calmodulin enhances the phosphatase and the estrogen binding activities of EBP by neutralizing the activity of Ca²⁺⁺ ions.

These preliminary observations suggest that Calmodulin enhances estrogen binding of EBP by sequestering Ca²⁺⁺ ions while calmodulin enhances the estrogen binding activity of the classical ER by phosphorylation in which Ca²⁺⁺ ions are required. It is clear from these results that calmodulin enhances estrogen binding of EBP as well as classical ER by two diametrically opposed mechanisms. A role for estrogen and for the phosphatase activity of EBP may be of importance in Alzheimer's disease which has been shown to be in steady increase in the aging population (73-74). Interestingly, the disease is 3 times more common in postmenopausal women than in older men and this has been related to the decrease in the availability of estrogen in women. The rationale provided for the variations in the occurrence of the disease in women and men is that since estrogen is metabolized from the androgens which are synthesized at all ages of men, estrogen appears to be available to men throughout their life while this is not the case in postmenopausal women. Presently, there is no conclusive evidence available to delineate the causal relationship between the estrogen protective action against the development of Alzheimer's disease in spite of the above plausible explanation. This is primarily due to a lack of an understanding of the disease as well the unavailability of an appropriate experimental animal model. The present understanding of Alzheimer's disease is that it can be characterized by the formation of senile plaques and neurofibrillary tangles which probably leads to neuronal death. Putative Alzheimer disease specific proteins (A68) have been purified and shown to be the major subunit of one of the paired helical filaments. The amino acid sequence and immunological property indicate that the backbone of A68 is indistinguishable from that of the protein **tau** which in the Alzheimer's disease is hyperphosphorylated. The "**lys-ser-pro-val**" motives in which the serine is the phosphate acceptor site is distributed in the Tau protein. There is strong evidence to conclude that excessive or inappropriate phosphorylation contributes to the formation of paired helical filament tangles (75-77). This hypothesis has been tested in an in vivo model to study Alzheimer's disease. A group of German investigators induced hyperphosphorylation of the tau protein and of neurofilaments of the microtubule by meticulously injecting stereotaxically the potent phosphatase inhibitor okadaic acid, resulting in the redistribution of large projection neurones from the axonal compartment into the cell bodies where they appeared as paired helical filament tangles similar to those seen in Alzheimer's disease (78-81). This model can be used to investigate whether EBP and estrogen can effectively counteract such an action and prevent the development of Alzheimer's disease. The present finding of phosphatase activity of EBP can be used to synthesize potent compounds which selectively increase the phosphatase activity of EBP to combat this disease.

Calcineurin is also known to be involved in the immunosuppression action of cyclosporin and FK506 (82). Both the immunosuppressants function by acting as prodrugs, only becoming active when complexed to endogenous, intracellular receptors or binding proteins named immunophilins. The target of the complex between the drug and its cognate immunophilin is the protein phosphatase calcineurin (83). Such is the case for cyclosporin when bound to its receptor cyclophilin (84,85), for FK506, when bound to its receptor, FKBP (86-90) and rapamycin when bound to FKBP (83). Great strides have been made in understanding the basic immunopharmacology of the immunosuppressants and in demonstrating the effective use of these agents not only in human transplantation but also in combating autoimmune disorders (91-93). Recently immunophilins have been shown to mediate the neuroprotective effects of FK506 in focal cerebral ischaemia (94). In this context it is worth recalling the reported incidences of cerebral haemorrhage and focal cerebral ischaemia with the use of high dose synthetic estrogens used in the contraceptive pills in the early developmental stages. All these various aspects of immunosuppressants may be relevant and of interest in the light of the observed similarities between EBP and calcineurin.

It must be pointed out that an indirect classical ER involvement in the immunosuppression action of FK506 has also been proposed based on similarities found between some peptides of cyclophilin and the heat shock protein p59, which interacts with the classical ER. Cyclophilin interacts with p59 heat shock protein (95). There is no information published on a direct interaction between an estrogen binder such as EBP and the immunosuppressants. Additional supportive evidence to confirm these observations can readily be obtained with the use of radiolabelled immunosuppressants. In any event the observation that EBP may play a role in the immunosuppressive action of these agents itself is most interesting and opens a variety of channels for further development of safe and potent new immunosuppressive agents.

### A role for EBP in central nervous system and behavior:

There is growing awareness that the rapid effects of steroid hormones in the central nervous system, on bone cells, smooth muscle, blood, liver, uterus, sperm, heart and pituitary can not be explained on the basis of effects induced at the genomic level. Thus these effects have to be due to non-genomic effects (96-101). This hypothesis is more pertinent particularly with questions related to the functions of the central nervous system. There is considerable evidence available to demonstrate that estrogen regulates the release of some of the anterior and posterior pituitary polypeptide hormones (FSH, LH, Prolactin, Oxytocin, vasopresin). More importantly, estrogens have been reported to exert antidopaminergic effect at the striatal and pituitary level in that they decrease amphetamine-induced stereotypes, decrease the circuling behavior induced by apomorphine treatment, potentiate spiroperidol-induced catalepsy, block the dopamine induced increase in straited acetylcholine levels, decrease the haloperidol induced in straited acetylcholine levels, reverse the inhibitory effect induced by dopamine or its agonists on prolactin release and induce a beneficial effect on dyskinesias associated with a hyperfunction of dopaminergic transmission in the extrapyramidal system induced by chronic neuroleptic therapy or with L-Dopa (10-19, 102-113).

The strong relationship between the actions of dopamine and estrogen prompted us to determine whether dopamine influences ³H-estradiol-17β binding to EBP. Dopamine inclusion in the incubation did not significantly influence the ³H-estradiol-17β binding to EBP both at low and high molarity. Additionally, no binding of radiolabelled dopamine to EBP was observed. These results do not support a direct common entity for estradiol-17β and dopamine.

Since estrogen alleviates psychosis, some commonly used antipsychotic agents were tested (pimozide, penfluridol, haloperidol, trifluoperazine, risperidone) in low and high concentrations for their ability to inluence ³H-estradiol-17β binding to EBP. Interestingly, the antipsychotic agents significantly increased the number of ³H-estradiol-17β binding sites on EBP in low concentrations and inhibited the estrogen binding at high concentrations. The preliminary in vitro competitive potency determination of antipsychotic agents with estrogen for EBP binding sites appears to parallel the in vivo antipsychotic potency. Confirmation of these results and establishment of a correlation of the in vitro results with any of the functional biological effect of estrogen, such as for example the regulation of prolactin release in a bioassay, will be most helpful to understand the possible common mechanism of action of estrogen and the antipsychotic agents. Interestingly, the majority of antipsychotic agents regulate prolactin level. Establishment of a correlation between the in vitro competitive potency of antipsychotic agents and the in vivo biopotency will be helpful to delineate a role and the importance of EBP in the central nervous system. It is clear that such observations are ultimately useful in developing potent new antipsychotic drugs.

### MECHANISM OF ACTION OF ENVIRONMENTAL ESTROGENS (XENOESTROGENS) VIA EBP:

Presently, a large number of varieties of pesticides, drugs, fuels and plastics have become increasingly prevalent in especially the western world and are distributed in enormous quantities in the waters of many nations. These compounds have long half lives (some as much 100 yrs) and are very difficult to biodegrade. Scientists have attempted to correlate the distribution of pollutants with an increase in a number of anomalies found in nature and have repeatedly, warned for the suspected effect of environmental estrogen pollutants (xenoestrogens). The decrease in wildlife and fish population, the increase in the number of male new borns with malformed genitals, increase in the incidence of human testicular and prostate cancers and a reduction in the sperm production in men compared to the past decades have been suspected to be due to the estrogenic action of some of the pollutants (114). Negligence of this potential problem due to insufficient information may have devastating consequences. Precedence of one such calamity in the past is the vaginal cancer in female children of mothers who used diethylstilbestrol during pregnancy (115,116). In this regard, we and others have pointed out that citral, a common additive in orange juice and perfumes, has an estrogenic action and induces prostatic hyperplasia in animals (117,118). Geraniol, a precursor of citral, an additive in embalming cream has been associated with gynaecomastia in men who perform the embalming (119). The in vitro estrogenic potency of all these compounds may not correlate with their in vivo potency as well as their detrimental action(s). Although, some of the compounds were known to have an estrogenic action in animal models they were approved in the past by the regulating agencies because of their low or undetectable estrogenic potency and were interpreted as having either no or acceptable weak deleterious effects on humans. Furthermore, there are hardly any investigations on long term cumulative effect of these compounds. This situation can be corrected only if it can be demonstrated with experimental evidence that some of the pollutants are potent long acting xenoestrogens and some of them may even be carcinogens.

In the light of the above information attempts were made to analyse whether some of the known pollutants and carcinogens (1-OH, 3-OH, 8-OH, 10-OH benzo(a)pyrene, 7,12-dimethylbenzanthracene (DMBA) and nonylphenol at 1, 10 and 100 pmol) compete in vitro with ³H-estradiol-17β for binding site on EBP. All the tested compounds competed with ³H-estradiol-17β to a significant extent. The order of the in vitro estimated relative potency for the diverse benzo(a)pyrenes were as follows: **8-OH > 3-OH >1-OH >10-OH compounds**. Since benzo(a)pyrene is a known carcinogen it will be important to determine whether there is a structural relationship between estradiol-17β and the various hydroxy benzo(a)pyrenes. DMBA and nonylphenol also reduced significantly ³H-estradiol-17β binding to EBP. Analysis of competitive action of DMBA using a sucrose gradient centrifugation technique clearly showed an inhibition of ³H-estradiol-17β binding by DMBA (Fig. 17). It is well known that DMBA is a potent carcinogen and as such it has been extensively used to induce estrogen dependent mammary and endometrial cancers in animals. Importantly, estrogen has been shown to enhance while progesterone to delay the time of tumor induction by DMBA. Nonylphenol has been shown to be estrogenic but to date there is no definitive evidence to consider it as a carcinogenic agent. However, this agent is still utilized to coat common plastics of daily use. This is the first demonstration of interaction of more than one known carcinogen with an estrogen binder (EBP). Establishment of a correlationship between the in vitro and in vivo potency of such compounds will provide a rapid in vitro screening assay. Most importantly, such information for the first time will delineate a definitive role for an estrogen binder such as EBP in carcinogenesis and may also enable to explain why compounds such as DMBA induce tumors in estrogen responsive tissues as well as why the tumors are estrogen dependent. Presently, without any reservations, such demonstration and information are direly needed and may help not only to explain the steady increase in the incidence of cancers but also to combat the disease.

### DETECTION OF EBP USING IMMUNO-CYTO AND -HISTOCHEMICAL TECHNIQUE:

Investigations of detection of EBP using immunochemical analysis in various cells and organs showed interesting information.

### EBP in bone cells:

EBP was detected on the cell and nuclear membrane of normal osteoblasts and osteosarcoma cells (Figs. 7 and 8) while the controls in which the pre-immune sera was used as first antibody and when the first antibody was not included in the procedure showed no staining.

### EBP in breast cancer:

Immuno-cytochemical analysis of human estrogen dependent breast cancer cells (MCF-7) showed that both the cytoplasm and the nuclei were stained for EBP (Figs. 9 & 10). The analysis of human breast cancer tissue (sections of fresh frozen and parafin) showed the localization of EBP in the glandular cells (Fig. 11); but what appeared to be non-specific staining was also observed in a number of other areas of the tumor. The non-specific staining seen in tumor tissue may be due to a number of reasons including the method of tissue preparation and fixation, the use of non-optimal high concentration of polyclonal first antibody or the non-specific nature of the second antibody. Additionally, the endogenous peroxidase may also contribute the non-specific staining. The reasons for non-specific staining will be investigated further.

### EBP in brain:

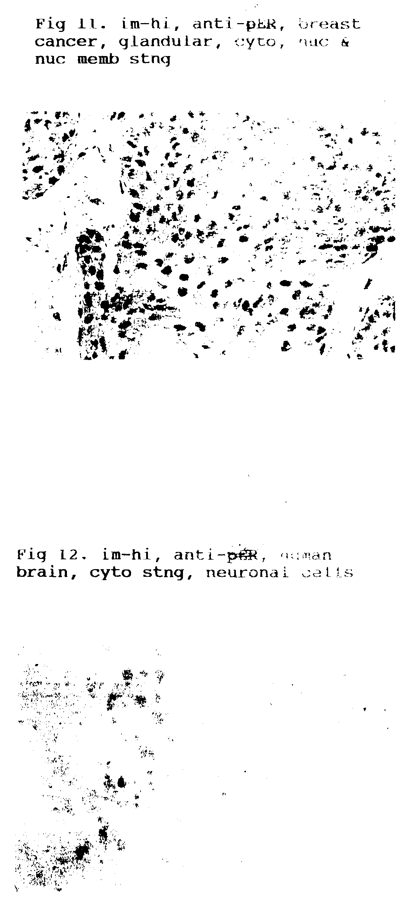

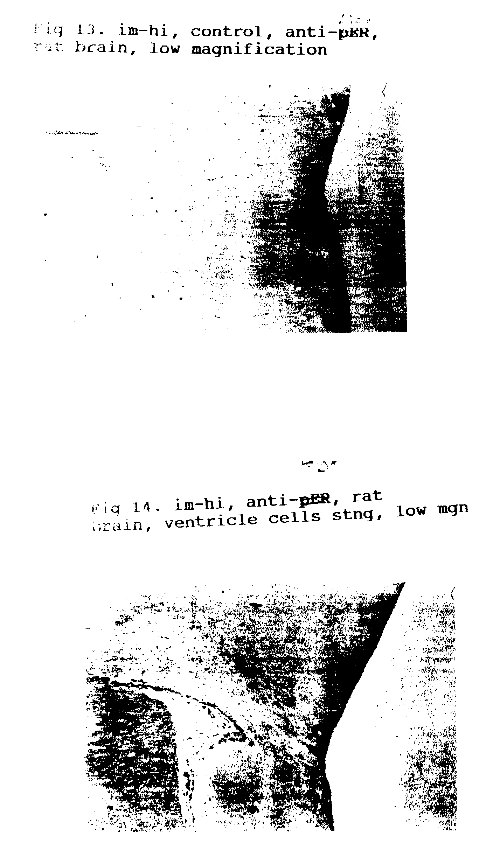

Analysis of a few sections of human brain showed cytoplasmic staining of neuronal cells (Fig. 12) while rat brain showed surprisingly clear staining of cells around the ventricle (ependymal cells ?) and a track of neuronal cells that appears to be of hippocampal region (Figs. 13 to 16).

### OTHER IMMUNOHISTOCHEMICAL ANALYSES PERFORMED:

A number of other tissues were also tested for the presence of EBP using immuno-histochemical technique. Such analyses showed interesting information which may be of importance.

### EBP in human benign prostatic hyperplasia:

Animal experiments have suggested that estrogen has a role in benign prostatic hyperplasia (BPH). However, to date the attempts made to demonstrate the definitive presence of classical ER in human BPH have not been successful. The few published reports of the presence of ER in human BPH by one or two groups have not been reproduced or confirmed. The presence of ER in the human BPH is still debatable.

Human BPH in parafin sections showed a selective staining of the glandular epithelial cells of the acini for EBP in the immuno-histochemical analyses while the stromal cells were clear of staining. Relatively, a large number of acinii was seen in each of the sections. These observations support the contention that human BPH is mainly due to the proliferation of glandular cells. Additionally, they support the hypothesis of an estrogenic role in BPH. More importantly, the present observation suggest that the estrogenic action may be mediated by EBP.

### EBP in bone osteoblast and osteoclast cells:

Immuno-histochemical staining of parafin sections of the rat femur for the presence of EBP showed a selective staining of the membranes (cell and nuclear) as well as in the nuclei of osteoblasts. A similar but clearly less dense staining pattern was observed in what appeared to be osteoclast cells. The presence of a meager amount of classical ER in the osteoblasts has been reported by a number of groups (120-121). However there is both apprehension as well as controversy regarding the classical ER role in the estrogenic action on osteoblasts (25-29, 122). The detection EBP in the bone cells opens another avenue for the delineation of the mechanism by which estrogen induces its action on bone cells.

### EBP in hair follicle:

Estrogens have been suspected to a play role in the hair growth (123-125). The analyses of parafin sections of mouse facial whiskers exhibited some exceptional picture. Interestingly, bulb papilla cells of whiskers (mouse) were selectively stained for the presence EBP. The bulb papilla cells were clustered in what appears to be small bundle of cells around a non-stained central core of the hair shaft. This staining pattern is seen in all the facial whiskers. This observation may be considered to support the contention that estrogen may play a role in hair growth regulation. Cyclosporin treatment prolongs the hair existing phase of the hair cycle in nude mice (126). It may be recalled that immunosuppressant agents cyclosporin and FK506 interact with EBP. This reasoning again lends credence to the suggestion that the hair growth regulatory role of estrogen and the effect of the prolongation of existing hair by immunosuppressants is mediated via EBP.

### EBP in eye lens:

Relatively few publications are available on whether steroid hormones and particularly estrogens have a role in the crystalline lens of the eye. The available information has suggested a role for estradiol in the epithelial cells under the lens capsule and this is mediated via a regulation of derivatives of lipoxygenase (127,128). Another study has suggested a modest protective effect of estrogen exposure on the lenses of women (129). More information on this aspect may be soon available since the introduction of estrogen replacement therapy in postmenopausal women.

### ESTROGEN AND SKIN:

The role of estrogen in the female skin has been of interest in the context of wrinkling and withering of the skin in postmenopausal women. This status of the skin has been related to loss of collagen. Both collagen and skin have an unique amino acid, i.e a proline in which the hydroxyl has been transformed from the cis to trans position. This is nescessary for the proline to be incorporated in the collagen chain. This area has been intensively investigated and it has been repeatedly demonstrated that the enzyme peptidyl-prolyl cis-trans isomerase (PPIase) executes the transformation of the hydroxyl from cis to trans position on proline (130-134). Most interestingly, PPIase and cyclophilin (both about 17 KD proteins) which are present in different isoforms have been shown to be closely related (135). In this connection it is important to recall that B subunit of EBP frequently migrates in the gel electrophoresis as a 17 KD polypeptide, estrogen binding of EBP is dependent on the presence of the B subunit, cyclosporin A influences estradiol-17β binding to EBP. All these facts suggest that it is possible that the EBP, B subunit, may also show PPIase activity and estrogen may either regulate its level as well as its activity. If this were to be the case, then, it may enable to explain the importance of estrogen in both bone building and the maintenance of the skin collagen in which PPIase plays a critical role.

All the above suggests that EBP estrogenic action may be involved in normal neural, vascular, immune and skeletal systems. In addition EBP may be involved in estrogen dependent cancers. Estrogenic actions mediated through EBP appear to complement the hitherto known mechanisms of estrogen action mediated via the classical ER (type I and II) and may now explain the estrogenic actions in full and in various physiological systems.

**Table 1**

| **COMPARISON OF PROPERTIES OF THE CLASSICAL ESTROGEN RECEPTOR (ER) AND THE NEW PUTATIVE ESTROGEN RECEPTOR ER.** | | |
|---|---|---|
| **Property** | **ER** | **EBP** |
| 1. Localization | cytoplasm & nucl. | dependent on cell type cell & nuclear membrane, cytoplasm & nucleus |
| 2. Molecular weight | 66,000 dalton | 84,000 dalton |
| 3. sulfhydryl agent | enhances binding | no difference |
| 4. Dissociation constant | about 1 x 10⁻¹⁰ M two binding sites, high affinity saturable, low affinity high capacity | about 0.7 x 10⁻¹⁰ M single binding site, cooperative binding effect exists, saturable |
| 5. Specificity | most estrogens Des binds no competition with (prog, androgen & corticoids) | most estrogens no Des binding no competition (prog, androgen, corticoids) |
| 6. Phosphorylation amino acid | enhances binding tyrosine | dephosphorylation enhances binding serine |
| 7. Heat shock protein | interacts | no interaction |
| 8. Phosphatase activity | no | yes, inherent serine phosphatase |
| 9. Neuroleptics | no effect/unknown | enhances binding at low concentration, inhibits at high concentration |
| 10. Calmodulin | interacts/binds | no interaction enhances binding |
| 11. Organs | most of estrogen responsive, vagina no def. info | most of estrogen responsive, no info on vagina |
| 12. α₂-anti-plasmin | no interaction | shares epitope |
| 13. Calcineurin | no interaction | shares epitope |
| 14. Anti-ER | interacts | no interaction |
| 15. Anti-EBP | no interaction | interacts |
| 16. Estrogen binds to ER-anti-ER complex | yes | - |
| 17. Estrogen binds to EBP-anti-EBP | - | no |
| Estrogen binding was evaluated using ³H-estradiol-17β and ³H-diethylstilbestrol. charcoal assay and sucrose density gradient centrifugation techniques were used to separate the bound from unbound steroids. | | |

### Figure Legend.

Fig. 1. Gel electrophoresis (8-25% gel) of a sample in the final phase of purification run on a gel filtration column (soporose 200). Peak fractions collected were electrophoresed and stained with silver staining. Bands in the 82-84 KD and 61-67 KD ranges are seen.
Fig. 2. Gel electrophoresis (8-25% gel) of a purified sample of the 61-67 KD range showing the presence of more than a single protein in the fraction.
Fig. 3. A purified sample (82-84 KD) treated with sodium dodocyl sulfate and β-mercaptoehtanol was run on gel electrophoresis (8-25% gel). A single band of the reduced sample migrated in the 61-65 KD range can be seen.
Fig. 4. EBP samples run on gel electrophoresis were transferred onto cellulose nitrate and reacted with antibodies. (left) anti-EBP, (right) anti-ER. Only the anti-EBP cross reacted while there was no cross reaction with anti-ER. Similar experiment with standard ER showed no cross reaction with anti-EBP (figure not shown).
Fig. 5. Pure EBP samples run on gel electrophoresis were transferred onto nitrocellulose membrane. The membrane was reacted with **anti-α**_{**2**}**-antiplasmin-plasmin complex.** (left) purified EBP containing 82-84 and 61-67 KD bands, (right) standard **anti-α**_{**2**}**-antiplasmin-plasmin complex.** The **anti-α**_{**2**}**-antiplasmin-plasmin complex** was provided by Prof.Lijnen, Leuven, Belgium. The EBP clearly cross reacts with **anti-α**_{**2**}**-antiplasmin-plasmin complex**. Similar experiment performed with anti-EBP showed cross reactivity with EBP as well as **anti-α**_{**2**}**-antiplasmin-plasmin complex.**
Fig. 6. Sulfhydryl group reduced samples of EBP and authentic calmodulin (about 21 KD) were run on gel electrophoresis (8-25% gel) and transferred onto nitrocellulose membrane. The membrane was reacted with anti-calmodulin. Only the authentic calmodulin in the two center lanes cross reacted while the there was no cross reaction in the other lanes containing EBP sub-units.

### IMMUNNOCYTO AND -HISTOCHEMICAL STAINING.

The standard DAB staining using the well established procedure was followed in performing the immuno-histochemical staining. The control samples were either not treated with the anti-EBP or treated with normal pre-immune serum. Some brain sections from human and rat were also treated with microwave in order to study the effect. Staining was not discernable in the microwave treated samples.
Fig. 7. Immuno-histochemical staining with anti-EBP (82-84 KD) of normal mouse osteoblast cells. Control performed with no anti-EBP in the reaction,
Fig. 8. Immuno-histochemical staining with anti-EBP (82-84 KD) of the mouse osteoblast. Cell membrane staining is seen in the cells in which anti-EBP has reacted.

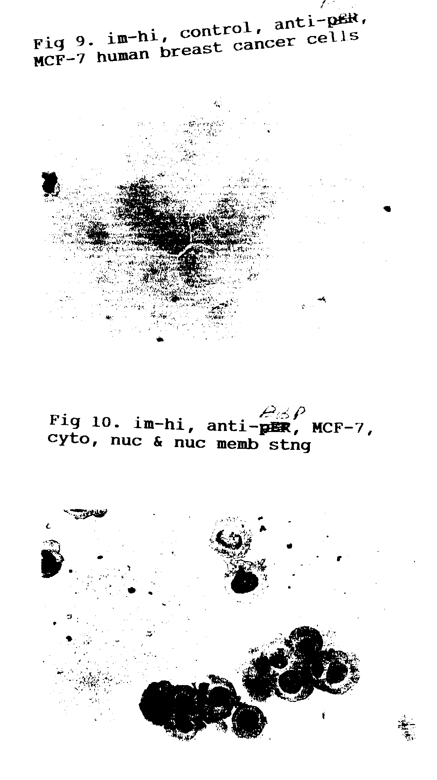

Fig. 9. Estrogen dependent MCF-7 cells from human breast cancer (obtained from TNO and AvLH) was treated following fixation with anti-EBP (82-84 KD) and DAB stained. Control performed with no anti-EBP included in the reaction.
Fig. 10. Estrogen dependent MCF-7 cells from human breast cancer (obtained from TNO and AvLH) was treated following fixation with anti-EBP (82-84 KD) and DAB stained. Cells showing staining in the cytoplasm, nucleus and the nuclear membrane are seen.
Fig. 11. Section from fresh frozen human breast cancer (dept of pathology, AZVU) treated with anti-EBP (82-84 KD). Staining of the cytoplasm, nucleus and nuclear membrane in the mammary gland cells are seen.
Fig. 12. Section from frozen human brain (obtained from Brain Institute) treated with anti-EBP (82-84 KD). Cytoplasmic staining of the neuronal cells are seen.
Fig. 13. Section from frozen rat brain (obtained from Brain Institute) treated with anti-EBP (82-84 KD). Control sample radiated with microwaves. Low magnification.
Fig. 14. Section from frozen rat brain (obtained from Brain Institute) treated with anti-EBP (82-84 KD). Cells around the ventricle and a clear track of cells are stained. Low magnification.

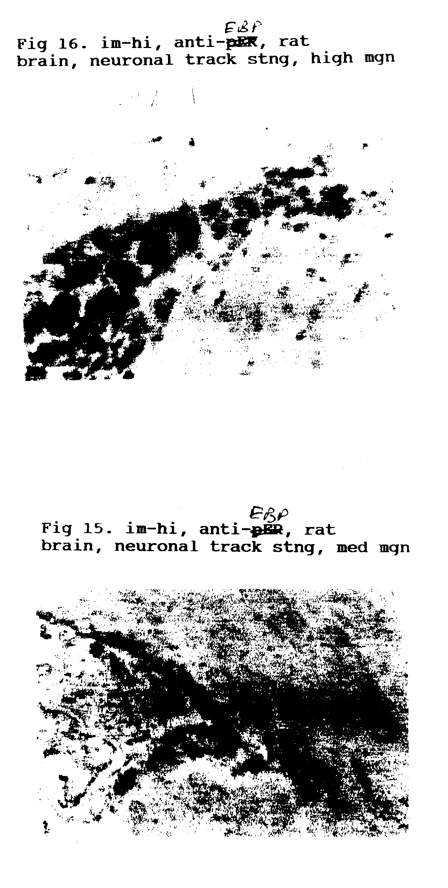

Fig. 15. Same section as in fig. 14. Medium magnification to show stained cell track.
Fig. 16. Similar section as in fig 14, to show the nuclear staining of neuronal cells forming a track (problem of focussing was encountered due to the thickness of the section). High magnification.

### Sucrose gradient centrifugation.

Fig. 17. Sucrose density gradient centrifugation (16 hrs, at 175,000 x g) profiles of EBP samples incubated with radiolabelled estradiol-17β and the carcinogen dimethylbenzanthracene in low and high concentrations. ³H-estradiol-17β (0.85 pmol) alone (_-----_), ³H-estradiol-17β (0.85 pmol) + dimethylbenzanthracene (0.42 nmol) (*------*) and ³H-estradiol-17β (0.85 pmol) + dimethylbenzanthracene (1.2 nmol) (+---+).

### References.

1. Walters MR. Steroid hormone receptors and the nucleus. Endocrine Reviews 6: 512-543, 1985.
2. Truss M, Beato M. Steroid hormone receptors: Interaction with deoxyribonucleic acid and transcription factors. Endocrine Reviews 14: 459-479, 1993.
3. Ciocca DR, Vargas Roig LM. Estrogen receptors in human nontarget tissues: Biological and clinical implications. Endocrine Reviews 16: 35-62, 1995.
4. White MM, Zamudio S, Stevens T, Tyler R, Lindenfeld J, Leslie K, Moore LG. Estrogen, progesterone, and vascular reactivity: Potential cellular mechanisms. Endocrine Reviews 16: 739-751, 1995.
5. Jensen EV, Suzuki T, Kawashima T, Stumpf WE, Jungblut PW, DeSombre ER. A two step mechanism for the interaction of estradiol with rat uterus. Proc Natl Acad Sci USA 59:632 1968.
6. Jensen EV. Intracellular localization of estrogen receptors: implications for interaction mechanism. Lab Invest 51: 487- , 1984.
7. O'Malley BW, Means AR. The mode of action of the female sex steroids. In Rickenberg EV (ed) Biochemistry of hormones. University press, London, Vol VIII: 197-, 1974.
8. Jensen EV, DeSombre ER. Mechanism of action of the female sex steroids. Annu Rev Biochem 41: 203-,1972.
9. O'Malley BW, Means AR. Female steroid hormones and target cell nuclei. Science 183: 610-, 1974.
10. Bedard P, Dankova J, Boucher R, Langelier p. Effect of estrogens on apomorphine-induced circling behavior in the rat. Can J Physiol Pharmacol 56: 538-541, 1978.
11. Bedard P, Langelier P, Dankova J, Villeeeuve A, Di Paolo T, Barden N, Labrie F, Boissier JR, Euvrard C. Estrogens, progesterone and the extrapyramidal system. In Extra-pyramidal system and its disorders. Advances in neurology series. (ed) LJ Poirier, TL Sourkes, P Bedard. Raven press, New York 1979, pp 411-422.
12. Bedard P, Langelier P, Villeneuve A. Estrogens and extrapyramidal system. Lancet 2: 1367-1368, 1977.
13. Euvrard C, Labrie F, Boissier JR. Effect of estrogen on changes in the activity of striatal cholinergic neurons induced by DA drugs. Brain Res 169: 215-220, 1979.
14. Annunziato L, Moore KE. Prolactin in CSF selectively increases dopamine turnover in the median eminence. Life Sci 22: 2037-2042, 1978.
15. Eikenburg DC, Ravitz Aj, Gudesky GA, Moore KE. Effects of estrogens on prolactin and tuberoinfundibular dopamine neurons. J Neural Transm 40: 235-244, 1978.
16. Labrie F, Beulieu M, Caron M, Raymond V. The adenohypophyseal dopamine receptors, specificity and modulation of its activity by estradiol. In Proceedings of Intl Symp on Prolactin. (ed) C Robin, M Harter, Elsvier/North Holland, Amsterdam, 1978, pp 121-136.
17. Lieberman ME, Maurer RA, Gorski J. Estrogen control of prolactin synthesis in vitro. Proc Natl Acad Sci, USA 75: 5946-5949, 1978.
18. Nicoll, CS, Meiters J. Prolactin secretion in vitro. Effects of gonadal and adrenal cortical steroids. Proc Soc Exp Biol Med 117: 579-583, 1964.
19. Raymond V, Beulieu M, Labrie F, Boissier JR. Potent antidopaminergic activity of estradiol at the rat pituitary level on prolactin release. Science 200: 1173-1175, 1978.
20. Power RF, Lydon JP, Conneely OM, O'Malley BW. Dopamine activation of an orphan of the steroid receptor superfamily. Science 252: 1546-1548, 1991.
21. Becker RC. Coronary artery disease in women: risks and prevention. Coronary Acute Care 3: 2-10, 1992.
22. Bush TL. The epidemiology of cardiovascular disease in postmenopausal women. Ann NY Acad Sci 592: 263-271, 1990.
23. Henderson BE, Ross RK, Paganini-Hill A, Mack TM. Estrogen use and cardiovascular disease. Am J Obstet Gynecol 154: 1181-1186, 1986.
24. Stampfer MJ, Colditz GA, Willett WC, Manson JE, Rosner B, Speizer FE, Hennekens CH. Postmenopausal estrogen therapy and cardiovascular disease. Ten year follow-up from the nurses' health study. N Eng J Med 325: 756-762, 1991.
25. Horowitz MC. Cytokines and estrogen in bone. Antiosteoporotic effects. Science 260: 626-627, 1993.
26. Girasole G, Jilka RL, Passeri G, Boswell S, Boder G, Williams DC, Manolagas SC. 17-estradiol inhibits interleukin-6 production by bone marrow-derived stromal cells and osteoblasts in vitro. J Clin Invest 89: 883-891, 1992.
27. Pacifico R, Brown C, Puscheck E, Friedrich E, Slatopolsky E, Maggio D, McCracken R, Avioli LV. Effect of surgical menopause and estrogen replacement on cytokine release from human blood mononuclear cells. Proc Natl Acad Sci 88: 5134-5137, 1991.
28. Pacifico R, Rifas L, McCracken R, Vered I, McMurtry C, Avioli LV, Peck WA. Ovarian steroid treatment blocks a postmenopausal increase in blood monocyte interleukin 1 release. Proc Natl Acad Sci 86: 2398-2402, 1989.
29. Chaudhary LA, Spelsberg TC, Riggs BL. Production of various cytokines by normal human osteoblast-like cells in response to interleukin-1 and tumor necrosis factor-: Lack of regulation by 17-estradiol. Endocrin 130: 2528-2533, 1992.
30. McGuire WL. Steroid hormone receptors in breast cancer treatment strategy. Recent Progress in Hormone Research. 38: 135-156, 1980.
31. Hierowski M, Madon J. Influence of 20-methylcholanthrene on 17β-estradiol binding to 9.5S receptor molecule from rat uterus. Biochem Biophys Acta 170: 425-427, 1968.
32. Heizmann CW, Wyss HI. Comparison of the binding of 7,12-dimethylbenzanthracene and estradiol 17 in the calf uterus cytosol. Steroids 20: 499-514, 1972.
33. Brecher PI, Wotiz HH. Stereospecificity of the uterine nuclear hormone receptors. Proc Soc Exptl Biol Med 128: 470-473, 1968.
34. Toft D, Spelsberg TC. A distinction between 3 methylcholanthrene and estrogen binding in the uterus. Cancer Res 32: 2743-2749, 1972.
35. Keightley DD, Okey AB. Effects of dimethylbenzanthracene and dihydrotestosterone on estradiol-17 binding in rat mammary cytosol fraction. Cancer Res 33: 2637-2642, 1973.
36. Schneider SL, Vitants A, Morreal CE, Sinha DK, Dao TL. Estrogenic properties of 3,9-dimethylbenzanthracene, a potential metabolite of benzanthracene. J Natl Cancer Inst 57: 1351-1354, 1976.
37. Rao BR. Estrogen receptors are not involved in estrogenic action of bisdehydrodoisynolic acid. Recent progress in hormone research, Portland, USA, 1995.
38. Meyers CY Kolb VM Gass GH Rao BR Roos CF Dandliker WB. Doisynolic-type acids-uterotopically potent estrogens which compete poorly with estradiol for cytosolic estradiol receptors. J steroid Biochem 31: 393, 1988.
39. Wehling M (ed). "The Genomic and Non-Genomic Effects of Aldosterone. CRC Press, Boca Raton, 1995.
40. Clark JH, Markaverich BM, Upchurch S, Eriksson H, Hardin JW, Peck EJ, Jr., Heterogeneity of estrogen binding sites: Relationship to estrogen receptors and estrogen responses. Recent Prog Horm Res 36: 89-134, 1980.
41. Markaverich BM, Roberts RR, Alejandro MA, Johnson GA, Middleditch BS, Clark JH. Bioflavonoid interaction with rat uterine type II binding sites and cell growth inhibition. J Steroid Biochem 30: 71-78, 1988.
42. Horigome T, Golding TS, Quarmby VE, Lubahn DB, McCarty K Sr, Korach KS. Purification and characterization of mouse uterine estrogen receptor under conditions varying hormonal status. Endocrinol 121: 2099-2111, 1987.
43. Markaverich BM, Gregory RR. Preliminary characterization and partial purification of rat uterine nuclear type II binding sites. Biochem Biophys Comm 177: 1283-1290, 1991.
44. Pink JJ, Jiang SY, Fritsch M, Jordan VC. An estrogenindependent MCF-7 breast cancer cell line which contains a novel 80-kilodalton estrogen receptor-related protein. Cancer Res 55: 2583-2590, 1995.
45. Jensen EV, Greene GL, Closs LE, DeSombre ER, Nadji M. Receptors reconsidered: A 20-year perspective. Recent progress in Hormone Research 38: 1-40, 1982.
46. Wiman B, Collen D. Purification and characterization of human antiplasmin, the fast-acting plasmin inhibitor in plasma. Eur J Biochem 78: 19-26, 1977.
47. Collen D, Wiman B. Fast-acting plasmin inhibitor in human plasma. J Am Soc Hematol 51: 563-569, 1978.
48. Wiman B, Collen D. On the mechanism of the reaction between human α₂-antiplasmin and plasmin. J Biol Chem 254: 9291-9297, 1979.
49. Lijnen HR, van Hoef B, Beelen V, Collen D. Characterization of the murine plasma fibrinolytic system. Eur J Biochem 224: 863-871, 1994.
50. Clemmensen I, Thorsen S, Mullertz, Petersen LC. Properties of three different molecular forms of the α₂-plasmin inhibitor. Eur J Biochem 120: 105-112, 1981.
51. Spitzer WO, Lewis MA, Heinemann LAJ, Thorogood M, MacRae KD On behlaf Transnational Research Group on Oral Contraceptives and Health of Young Women. Third generation oral contraceptives and risk of venous thromboembolic disorders: an international case-control study. BMJ 135: 127-132, 1996.
52. Lewis MA, Spitzer WO, Heinemann LAJ, MacRae KD, Bruppacher R, Thorogood M on behalf of Transnational Research Group on Oral Contraceptives and Health of Young Women. Third generation oral contraceptives and risk of myocardial infarction: an international case-control study. BMJ 135: 132-134, 1996.
53. Gevers Leuven JA, Dersjant-Roorda MC, Helmerhorst FM, de Boer R, Neymeyer-LeLoux A, Havekes L. Estrogenic effect of gestodene- or desogestrel- containing oral contraceptives on oral lipoprotein metabolism. Am J Obstet Gynecol 163: 358-362, 1990.
54. Royal College of General Practitioners. Further analyses of mortality in oral contraceptive users. Lancet 1: 541-546, 1981.
55. Vessey MP, Villard-Mackintoch L, McPherson K, Yeates D. Mortality in oral contraceptive users. BMJ 199: 1487-1491, 1989.
56. Inman WHW, Vessey MP. Investigation of deaths from pulmonary, coronary and cerebral thrombosis and embolism in women of child-bearing age. BMJ 2: 193-199, 1968.
57. Mann JI, Inman WHW. Oral contraceptives and death from myocardial infarction. BMJ 2: 245-248, 1975.
58. Mann JI, Inman WHW, Thorogood M. Oral contraceptive use in older women and fatal myocardial infarction. BMJ 2: 445-447, 1975.
59. Adams SA, Thorogood M, Mann JI. Oral contraception and myocardial infarction revisited: the efffects of new preparations and prescribing patterns. Br J Obset Gynaecol 88: 838845,1981.
60. Jesperson J and Gram J. Increase fibrin formation in blood in women above the age of 30 who are both oral contraceptive users and smokers. Fibrinolysis 10: 9-13, 1996.
61. Klee CB, Krinks MH, Manalan AS, Cohen P, Stewart. Isolation and characterization of bovine brain calcineurin: A calmodulin-stimulated protein phosphatase. Methods in Enzymology 102: 227-244, 1983.
62. Auricchio F, Migliaccio, Casoria G, Rotondi A, Di Domenico M. Calmodulin-stimulated estradiol receptor-tyrosine kinase. Methods in Enzymology 139: 731-744, 1987.
63. Castoria G, Migliaccio A, Green S, Di Domenico M, Chambon P, Auricchio F. Properties of a purified estradiol-dependent calf uterus tyrosine kinase. Biochemistry 32: 1740-1750, 1993.
64. Arnold SF, Obourn JD, Jaffe H, Notides AC. Phosphorylation of the human estrogen receptor on tyrosine 537 in vivo and by Src family tyrosine kinases in vitro. Mol Endocrinol 9: 24-33, 1995.
65. Bouhoute A, Leclercq G. Antogonistic effect of triphenylethylenic antiestrogens of the association of estrogen to calmodulin. Biochem Biophys Res comm 184: 1432-1440, 1992.
66. Brautigan DL. Great expectations: Protein tyrosine phosphatases in cell regulation. Biochim Biophys Acta 1114: 63-77, 1992.
67. Walton KM, Dixon JE. Protein tyrosine phosphatases. Annu Rev Biochem 62: 101-120, 1993.
68. LaForgia S, Morse B, Levy J, Barnea G, Cannizzaro LA, Li F, Nowell PC, Boghosian-Sell l, et al., Receptor-protein-tyrosine phosphatse τ is a candidate tumor suppressor gene at human chromosome region 3p21. Proc Natl Acad Sci, USA 88: 50365040, 1991.
69. Cohen P. The structure and regulation of protein phosphatases. Annu Rev Biochem 58: 453-508, 1989.
70. Guan KL, Dixon JE. Science 249: 553-556, 1990.
71. Tonks CJ, Lai DSY, Chia HP, Boulet I, Tong PH. Biochem J 276: 315-323, 1991.
72. Fisher DK, Higgins TJ. A sensitive high-volume, colorimetric assay for protein phosphataes. Pharmac Res 11: 759-763, 1994.
73. Dillmann R. Alzheimer's disease. The concept of disease and the construction of medical knowledge. Thesis, Vrije Universiteit, Amsterdam, 1990.
74. Burns A, Howard R, Pettit. Alzheimer's disease: a medical companion, Blackwell Science, 1995, pp 25-153.
75. Lee VMY, Balin BJ, Otvos L Jr, Trojanowski JQ. A68: A major subunit of paired helical filaments and derived forms of normal Tau. Science 251: 675-678, 1991.
76. Paudel HK, Lew J, Ali Z, Wang JH. Brain proline-directed protein kinase phosphorylates Tau on sites that are abnormally phosphorylated in Tau associated with Alzheimer's paired helical filaments. J Biol Chem 268: 23512-23518, 1993.
77. Vulliet R, Halloran SM, Braun RK, Smith AJ, Lee G. Proline-directed phosphorylation of human Tau protein. J Biol Chem 267: 22570-22574, 1992.
78. Arendt T, Hanisch F, Holzer M, Bruckner. In vivo phosphorylation in the rat basal nucleus induces PHF-like and APP immunoreactivity. NeuroReport 5: 1397-1400, 1994.
79. Arendt T, Holzer M, Fruth R, Bruckner MK, Gartner U. Paired helical filament-like phosphorylation of Tau, deposition of β/A4-amyloid and memory impairment in rat induced by chronic inhibition of phosphatase 1 and 2A. Neuroscience (in press, personnel communication of Dr.Arendt).
80. Arendt T, Fruth R, Holzer M, Bruckner MK. Induction of Alzheimer-type pathology in rat brain by chronic stimulation of protein phosphorylation. The meeting of european neuroscience, Amsterdam, The Netherlands, 3-7 sept, 1995, abstract #12.03.
81. Lesort M, Couratier P, Esclaire F, Yardin C, Hugon J. Protein kinase C activation induces neuronal Tau phosphorylation at serine 202. The meeting of european neuroscience, Amsterdam, The Netherlands, 3-7 sept, 1995, abstract #50.37.
82. Liu J, Farmer Jr JD, Lane WS, Friedman J, Weissman I, Schreiber SL. Calcineurin is a common target of cyclophilincyclosporin A and FKBP-FK506 complexes. Cell 66: 807-815, 1991.
83. Schriber SL, Crabtree GR. The mechanism of action of cyclosporin A and FK506. Immunology Today 13: 136-142, 1992.
84. Handschumacher RE, Harding MW, Rice J, Drugge RJ. Science 226: 544-547, 1984.
85. Harding MW, Handschumacher RE, Speicher DW. Isolation and amino acid sequence of cyclophilin. J Biol Chem 261: 8547-8555, 1986.
86. Harding MW, Galat A, Uehling DE, Schreiber SL. Nature 346: 758-760, 1989.
87. Standaert RF, Galat A, Verdine GL, Schreiber SL. Molecular cloning and overexpression of the human FK506-binding protein FKBP. Nature 346: 671-674, 1990.
88. Siekierka JJ, Hung SHY, Poe M, Lin CS, Sigal NH. Nature 341: 755-757, 1989.
89. Maki N, Sekiguchi F, Nishimaki J, Miwa K, Hayano T, Takahashi N, Suzuki M. Complimentary DNA encoding the human T-cell FK506-binding protein, a peptidylprolyl cis-trans isomerase distinct from cyclophilin. Proc Natl Acad Sci, USA 87: 9231-9235, 1990.
90. Sigal NH, Dumont FJ. Cyclosporin A, FK-506, and rapamycin: Pharmacologic probes of lymphocyte signal transduction. Annu Rev Immunol 10: 519-560, 1992.
91. Meyers BD, Ross J, Newton L, Luetscher J, Perloth M. Cyclosporine-associated chronic nephropathy. N Engl J Med 311: 699-705, 1984.
92. Feutern G, Mihatsch MJ: For the International Kidney Biopsy Registry of Cyclosporine in Autoimmune Diseases. Risk factors for cyclosporine-induced nephropathy in patients with autoimmune diseases. N Engl J Med 326: 1654-1660, 1992.
93. Fathman CC, Myers BD. Cyclosporine therapy for autoimmune disease. N Engl J Med 326: 1693-1694, 1992.
94. Sharkey J, Butcher SP. Immunophilins mediate the neuroprotective effects of FK506 in focal cerebral ischaenia. Nature 371: 336-339, 1994.
95. Ratajczak T, Carrello A, Mark PJ, Warner BJ, Simpson RJ, Moritz RL, House AK. The cyclophilin component of the unactivated estrogen receptor contains tetratricopeptide repeat domain and shares identity with p59 (FKBP59). J Biol Chem 268: 13187-13192, 1993.
96. Wehling M (ed). The genomic and non-genomic effects of aldosterone. CRC Press, Boca Raton, 1995.
97. Lieberherr M, Gross B. Androgens increase intracellular calcium concentration and inositol 1,4,5-triphosphate and diacylglycerol formation via a pertussis toxin-sensitive G-protwin. J Biol Chem 269: 7217-7223, 1994.
98. Szego CM, Pietras RJ. Lysosomal functions in cellular activation: propagation of the actions of hormones and other effectors. Int Rev Cytol 88: 1-302, 1984.
99. Blackmore PF. Rapid non-genomic actions of progesterone stimulate Ca²⁺ influx and the acrosome reaction in human sperm. Cell Signal 5: 531-538, 1993.
100. Koening H, Fan CC, Goldstone AD, Lu CY, Trout JJ. Polyamines mediate androgenic stimulation of calcium fluxes and membrane transport in rat heart myocytes. Ciculation Res 64: 415-426, 1989.
101. Bression D, Michard M, LeDafniet M, Pagesy P, Peillon F. Evidence for specific estradiol binding site on rat pituitary membranes. Endocrinol 119: 1048-1051, 1986.
102. Villeneuve A, Langlier P, Bedard P. Estrogens, dopamine and dyskinesias. Can Psychiatr Assoc J 23: 68-70, 1978.
103. Euvrard C, Oberlander C, Boissier JR. Antidopaminergic effect of estrogens at the striated level. J Pharmacol Exper Ther 214: 179-185, 1980.
104. Chiodo LA, Caggiula AR. Alterations in basal firing rate and autoreceptor sensitivity of dopamine neurons in the substantia nigra following acute and extended exposure to estrogen. Europ J Pharmacol 67: 165-166, 1980.
105. Klaiber EL, Broverman DM, Vogel W, Kobayashi Y. Estrogen therapy for severe persistent depressions in women. Arc Gen Psychiatry 36: 550-554, 1979.
106. Gamboa ET, Isaacs G, Harter DH. Chorea associated with oral contraceptive therapy. Arch Neurol 25: 112-114, 1971.
107. Villeneuve A, Cazejust T. Cote M. Estrogens in tardive dyskinesia in male psychiatric patients. Neuropsychobiol 6: 145-151, 1980.
108. Coppen A, Kessel N. Menstruation and personality. Brit J Psychiat 109: 711-721, 1963.
109. Gordon JH. Modulation of apomorphine-induced stereotypy by estrogen: Time course and dose response. Brain Res Bulletin 5: 679-682, 1980.
110. Gordon JH, Gorski RA, Borison RL, Diamond BI. Postsynaptic efficacy of dopamine: Possible suppression by estrogen. Pharmacol Biochem Behavior 12: 515-518, 1980.
111. Chiodo LA, Caggiula AR, Saller CF. Estrogen potetiates the stereotypy induced by dopamine agonists in the rat. Life Sci 28 : 827-835, 1981.
112. Arnauld E, Dufy B, Pestre M, Vincent JD. Effects of estrogens on the responses of caudate neurons to microiontophoretically applied dopamine. Neuroscience Lett 21: 325-331, 1981.
113. De Kloet ER, Voorhuis TAM, Elands J. Estradiol induces oxytocin binding sites in rat hypothalamic ventromedial nuclues. Europ J Pharmacol 118: 185-186, 1985.
114. Soto AM, Justicia H, Wray JW and Sonnenshein C. Environ Hlth Perspec 92 :167-173, 1991.
115. Herbst AL, Anderson D. Sem Surg Oncol 6: 343-346, 1990.
116. Sharp GB, Cole P, Anderson D, Herbst AL. Cancer 66:2215-2220, 1990.
117. Geldof AA, Engel C, Rao BR. Urol Res 20:139-144, 1992
118. Servadio C, Abramovici A, Sandbank U, Savion M, Rosen M. Eur Urol 12: 195-200, 1986.
119. Abramovici A, Sandbank U. N Engl J Med 319:1157, 1988.
120. Eriksen EF, Colvard DS, Berg NG, Graham ML, Mann KG, Spelsberg TC, Riggs BL. Evidence of estrogen receptors in normal human osteaoblast-like cells. Science 241: 84-86, 1988.
121. Komm BS, Terpening CM, Benz DJ, Graeme KA, Gallegos KA, Korc M, Greene GL, O'Malley BW, Haussler MR. Estrogen binding, receptor mRNA, and biologic response in osteoblst-like osteosarcoma cells. Science 241: 81-84, 1988.
122. Slootweg MC, Ederveen AGH, Schot LPC, Schoonen WGEJ, Kloosterboer HJ. Oestrogen and progesterone synergitically stimulate human and rat osteoblast proliferation. J Endocrinol 133: R5-R8, 1992.
123. Arai A, von Hintzenstern J, Kiesewtter F, Schell H, Hornstein OP. In vitro effects of testosterone, dihydrotestosterone and estradiol on cell growth of human hair bulb papilla cells and hair root sheath fibroblasts. Acta Dermato-Venereologica 70: 338-341, 1990.
124. Kiesewetter F, Arai A, von Hintzenstern J, Schell H. Effects of testosterone, dihydrosestosterone and estradiol on growth of human hair oute root sheath keratinocytes in vitro. Arch Dermatol Res 283: 476-479, 1991.
125. Vaillant L, Callens A. Skin and esthetic alterations in menopause. Rep Humaine et Hormones 8: 203-208, 1995.
126. Hozumi Y, Imaizumi T, Kondo S. Effect of cyclosporin on hair-existing area of nude mice. J Dermatol Sci 7: 33-38, 1994.
127. Bisaria KK. Effect of estrogen on lens epithelium in the albino rat. Indian J Physiol Pharmacol 24: 357-360, 1980.
128. Guivernau M, Terragno A, Dunn MW, Terragno NA. Estrogens induce lipoxygenase derivative formation in rabbit lens. Invest Opthalmol Visual Sci 23: 214-217, 1982.
129. Klein BE, Klein R, Ritter LL. Is there evidence of an estrogen effect on age-related lens opacities? The Beaver Dam Eye Study. Arch Opthalmol 112: 85-91, 1994.
130. Fischer G, Bang H, Mech C. Biomed Biochim Acta 43: 1101-1111, 1984
131. Fischer G, Bang H. Biochim Biophys Acta 828: 39-42, 1985.
132. Lang K, Schmid FX, Fischer G. Nature 329: 268-270, 1987.
133. Lang K, Schmid FX. Nature 331: 453-455, 1988.
134. Bachinger HP. J Biol Chem 262: 17144-17148, 1987.
135. Fischer G, Wittmann-Liebold B, Lang K, Kiefhaber, Schmid FX. Cyclophilin and peptidyl-prolyl cis-trans isomerase are probably identical proteins. Nature 337: 476-478, 1989.

## Claims

1. A murine estrogen binding proteinaceous substance or a mammalian equivalent thereof, the murine substance comprising a subunit having a molecular weight of about 61-67 kD as analyzed by SDS-PAGE.

2. An estrogen binding proteinaceous substance according to claim 1, which is a heterodimer, whereby one subunit of the murine substance is the 61-67 kD proteinaceous substance and the other subunit is a 17-22kD proteinaceous substance, whereby the resulting heterodimer has a molecular weight of about 81-84 kD as measured by gel electrophoresis.

3. An estrogen binding heterodimer according to claim 2 having an iso-electric point of about 4.35.

4. An estrogen binding heterodimer according to claim 2 or 3 which shows a single peak at 4.8 S in a sucrose density gradient centrifugation.

5. An estrogen binding proteinaceous substance according to claim 2-4, comprising a further subunit of about 61-67kD respectively as measured by gel electrophoresis.

6. An estrogen binding proteinaceous substance according to any one of the aforegoing claims which retains estrogen binding activity in the presence of the reducing agent DTT.

7. An estrogen binding proteinaceous substance according to any one of the aforegoing claims which does not show specific binding to anti-estrogen receptor antibodies.

8. An estrogen binding proteinaceous substance according to anyone of the aforegoing claims further having serine phosphatase activity.
